# EUROPEAN PATENT APPLICATION

(11) **EP 1 582 212 A1**
(43) Date of publication of application: **05.10.2005**
(21) Application number: 03772926.6
(22) Date of filing: 30.10.2003
(51) Int. Cl.: A61K 31/7004, A61K 9/08

(54) **INTRAVITREALLY-INJECTABLE SOLUTION FOR THE TREATMENT OF VITREOUS HAEMORRHAGES**

(30) Priority: 13.12.2002 MX JL02000046
(71) Applicant: Jimenez Bayardo, Arturo, Jalisco 44290 (MX)
(72) Inventor: JIMENEZ BAYARDO, Arturo, Guadalajara, Jalisco-44290 (MX); SANCHEZ CASTELLANOS, Victoria, E., Guadalajara, Jalisco 44290 (MX); PADILLA MORONES, Miguel, Guadalajara, Jalisco 44290 (MX); GONZALEZ, Jaime, R., Guadalajara, Jalisco 44290 (MX); BAIZA DURAN, Leopoldo, Martin, Guadalajara, Jalisco 44290 (MX)
(74) Representative: Carpintero Lopez, Francisco
(86) International application number: PCT/MX2003/000093
(87) International publication number: WO 2004/054589

(57) **Abstract**

The present invention relates to an ophthalmic solution for the clarification of vitreous hemorrhages. More specifically, it relates to a pharmaceutically acceptable intraocular injectable solution, for the treatment of vitreous hemorrhages, whereby the reabsorption of such hemorrhage is encouraged. It enables the clarification of the vitreous hemorrhage in a significantly short period of time to allow for the timely diagnosis of the lesion and the repair of the damage the hemorrhage has caused to the vitreous body. The ophthalmic solution of the present invention is injected at least once into the vitreous humor of the patient in a therapeutically effective dose to obtain the desired result.

## Description

### BACKGROUND TO THE INVENTION

### 1. Field of the invention.

This invention relates to an injectable solution for ophthalmic application. More specifically, a pharmaceutical solution fit to be injected intraocularly in the vitreous body of the eye for the treatment of vitreous hemorrhages.

### 2. Prior Art.

Proliferative diabetic retinopathy, ocular traumas and other ophthalmic disorders sometimes lead to the rupture or leakage of the retinal blood vessels, with the resulting bleeding into the eye's vitreous humor. This fact is known in medicine as vitreous hemorrhage and is typically manifested as clouding or opacification of the vitreous humor. The prognosis for vitreous hemorrhage depends on the underlying illness in patients with diabetic retinopathy, as the base disease. The prognosis worsens for both the resolution of the vitreous hemorrhage and for the visual recuperation. According to statistics, each year there are around 450,000 cases of vitreous hemorrhages in the United States of America alone and the only available alternative until now for treating such hemorrhage is surgery.

Vitreous hemorrhage may be the ophthalmic sign of presentation of a large number of retinal diseases, as a major cause of sight loss. The vitreous hemorrhage is sometimes accompanied by tearing or separation of the retina, so it is important for such tearing or separation to be quickly diagnosed and repaired surgically. A delay in the diagnosis and repair of the tear or separation of the retina can cause irreversible damage to the photoreceptor cells of the retina in the region of the tear or separation that can lead to a reduction of the patient's sight.

Furthermore, failing to take care of the separation of the retina can lead to an additional vitreous hemorrhage and/or the formation of fibrous tissue at the site of the hemorrhage. This fibrous tissue formation can cause an undesirable fibrous connection between the vitreous body and the retina.

The standard procedure used to repair the tears or separations of the retina require the surgeon to be capable of observing through the vitreous humor to view the damaged region of the retina. When the vitreous hemorrhage has occurred, the presence of blood inside the vitreous humor can cause this to become cloudy and the surgeon is unable to see the retina through the vitreous body. Moreover, the present of blood inside the vitreous humor can significantly deteriorate the patient's sight through the affected eye and this problem would persist until the blood has been substantially or completely cleared.

According to related studies in this field, only around 22% of patients who have suffered a vitreous hemorrhage can regain their sight without any treatment at the end of one year after the vitreous hemorrhage occurred. In these cases, the prolonged period of one year it takes for the natural absorption of the vitreous hemorrhage, sufficiently to allow the transvitreous viewing of the retina, is detrimental to the patient's health because this causes a considerable delay in the diagnosis and treatment of a tear or separation of the retina. For this reason, waiting for natural vitreous clarification is on no account a recommendable option.

Furthermore, even when the vitreous hemorrhage is not accompanied by a tearing or separation of the retina, it is difficult to verify that this has not occurred as a result of the hemorrhagic clouding of the vitreous humor preventing the specialist from conducting the routine funduscopic examination of the retina.

Additionally, the standard procedure at present for removing the vitreous hemorrhage consists in doing a vitrectomy, which is highly specialist surgery whereby all or part of the vitreous body is removed from inside the eye and replaced with a transparent liquid to permit the surgeon to view the retina sufficiently to examine it and/or surgically repair the hemorrhage and any accompanying tear or separation of the retina.

However, in addition to the highly necessary skill to carry out the vitrectomy procedures, this surgery is associated with some significant disadvantages, risks and complications. For example, the act of removing the vitreous body can cause additional tears or separations of the retina, or result in additional hemorrhaging of the already weakened retinal blood vessels. Also, the patient upon whom a vitrectomy is practiced is exposed to a high risk of eye infections, which can even lead to blindness. Another drawback with this surgery is that it can bring about the development of cataracts in a short period of time, making additional cataract surgery necessary.

On top of the above drawbacks, it should also be mentioned that vitrectomies are very expensive and that the patient is exposed to excessive risks associated with systemic anesthesia.

There are other alternatives under experimentation for the treatment of vitreous hemorrhages, which propose the intravenous injection of urea or carbamide, and there is another consisting in injecting an enzyme (hyaluronidase). Although these two alternative options exists, according to reports in medical publications, Vitrase is still at an experimental stage and has been for some years, and the problem of some adverse reactions derived from its intravitreous application has not been overcome, hence its predicted efficacy in the treatment of vitreous hemorrhages has still not been proven in order to consider such enzyme as a reliable and safe alternative for the treatment of such hemorrhages. It is known, for example, that Vitrase causes a strong inflammatory reaction when injected, whereas the experimental model based on urea or carbamide, discussed here for purposes of comparison with the present invention, does not offer the same effectiveness as the new injectable solution proposed in this application.

### SUMMARY OF THE INVENTION

One object of the invention is to propose a new pharmaceutically acceptable injectable solution for significantly reducing the clarification time of vitreous humor clouded by a hemorrhage, whereby the timely examination and diagnosis of the lesion in the eye that has caused the vitreous hemorrhage could be carried out and thereby propose the right treatment.

Another object is to abbreviate the clarification time of the vitreous humor by applying an effective treatment for reabsorption of the vitreous hemorrhage in order to make it possible to do, if necessary, transvitreous surgical procedures typically used to repair the site of the hemorrhage and/or repair any accompanying tear or separation of the retina.

A further object of the present invention is to propose a method of intravitreous injection of an ophthalmic solution that can avoid the need for vitrectomy surgery, and with it the high costs, risks and drawbacks associated within this kind of surgical operation.

To fulfill the aforementioned objects, a pharmaceutically suitable ophthalmic solution is proposed, which is injected into the vitreous body of the eye to clarify the hemorrhagic cloudiness in an exceptionally short time.

This new alternative proposes a solution formulated chiefly by an essentially hyperosmotic substance, to be injected into the vitreous cavity of patients with vitreous hemorrhage to thereby encourage fast and effective reabsorption of the hemorrhage in order to be able to make a timely and accurate diagnosis of the background pathology, and to treat the underlying cause of the vitreous hemorrhage.

### DESCRIPTION OF THE DRAWINGS

Figure 1 is a graph showing the behavior of the vitreous hemorrhage (HV) in the first group of an experimental model conducted on rabbits in which the treatment of the hemorrhage by intravitreous injection was carried out using a placebo.
Figure 2 is a graph showing the behavior of the vitreous hemorrhage (HV) in the second group of an experimental model conducted on rabbits subjected to treatment with an intravitreous injection of a solution whose active ingredient is a urea or carbamide, called here AL025.
Figure 3 is another graph of the behavior of the vitreous hemorrhage (HV) in the third experimental group subjected to treatment with a new injectable solution.
Figure 4 is a comparative graph of the behavior of the vitreous hemorrhage (HV) in the three experimental models developed, which are represented in the foregoing figures.

### DETAILED DESCRIPTION OF THE INVENTION

The present invention is related to an ophthalmic solution to be applied by injection into the vitreous body of the eye of a patient that has suffered a vitreous hemorrhage. The new injectable solution has been formulated for the treatment of vitreous hemorrhages and its intraocular injection encourages the reabsorption of the hemorrhage in the vitreous body in an exceptionally short time, allowing the patient to be able to be checked and diagnosed in good time as regards the lesion that caused such hemorrhage.

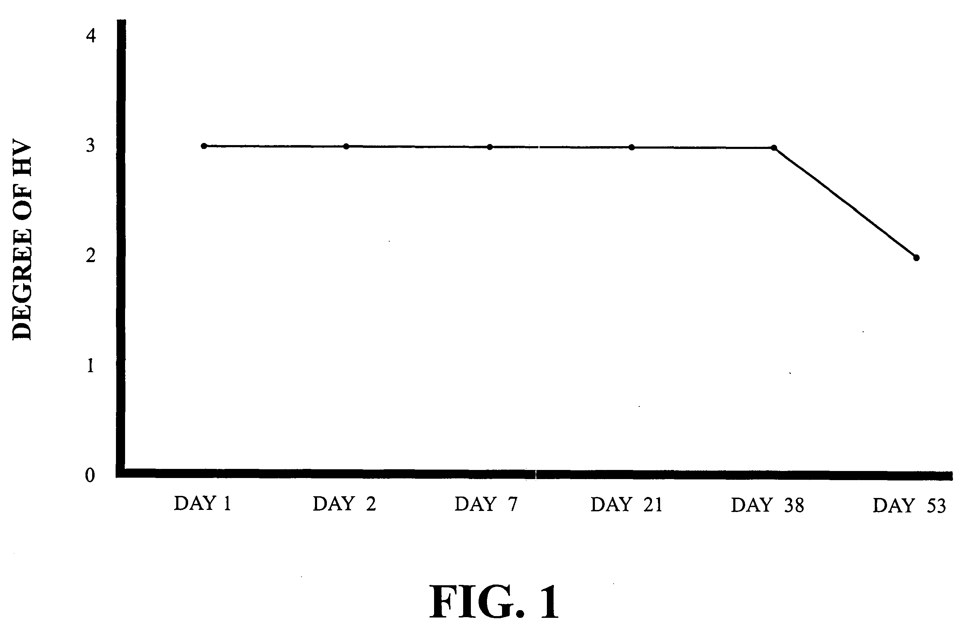

Figures 1 to 3 are graphs corresponding to respective experimental models carried out on rabbits upon which a hemorrhage in the vitreous body of only one of their eyes was induced. For this, 0.05 ml of autologous blood, without anticoagulant, from the dorsal vein and such blood was injected into the right eye. The following day, a clinical evaluation and assessment of the severity of the vitreous humor was conducted on them by indirect ophthalmoscopy. For purposes of this study, the following parameters of vitreous hemorrhage were established, according to the severity thereof:
HV 0 = No evidence of vitreous hemorrhage
HV+ = Vitreous hemorrhage that allows for detailed definition of the optic nerve and macula.
HV++ = Vitreous hemorrhage that does not allow definition of the details of the optic nerve and macula.
HV+++ = Vitreous hemorrhage that does not allow a view of the optic nerve and macula.

For all cases, it was decided to carry out the corresponding study for a period of 53 days, during which clinical assessments of the severity of the vitreous hemorrhage were conducted on days 1, 2, 7, 21, 38 and 53, in order to determine the behavior of the hemorrhage in the species subj ected to study.

Also, to carry out these studies, the seventh day after the induction of the vitreous hemorrhage was chosen on which to administrate the injection of the corresponding solution in each experimental model of figures 1 through 3. The aim of this was for the blood injected into the vitreous cavity of the rabbits to be able to disperse in the vitreous humor. However, it is important to mention that, unless there are contraindications, the injection can be applied to the patient at any time after the vitreous hemorrhage is diagnosed.

Thus, for the case of the model of figure 1, an intravitreous injection of a solution with no therapeutic efficacy (placebo) was applied to the right eye of the rabbits. As the graph shows, the behavior of the vitreous hemorrhage stayed practically uniform until the end of the study period. At the most, a slight decrease in the degree of the hemorrhage was recorded as of day 38 of such period. This reduction, however, was not sufficient to obtain favorable conditions for the viewing of the retina.

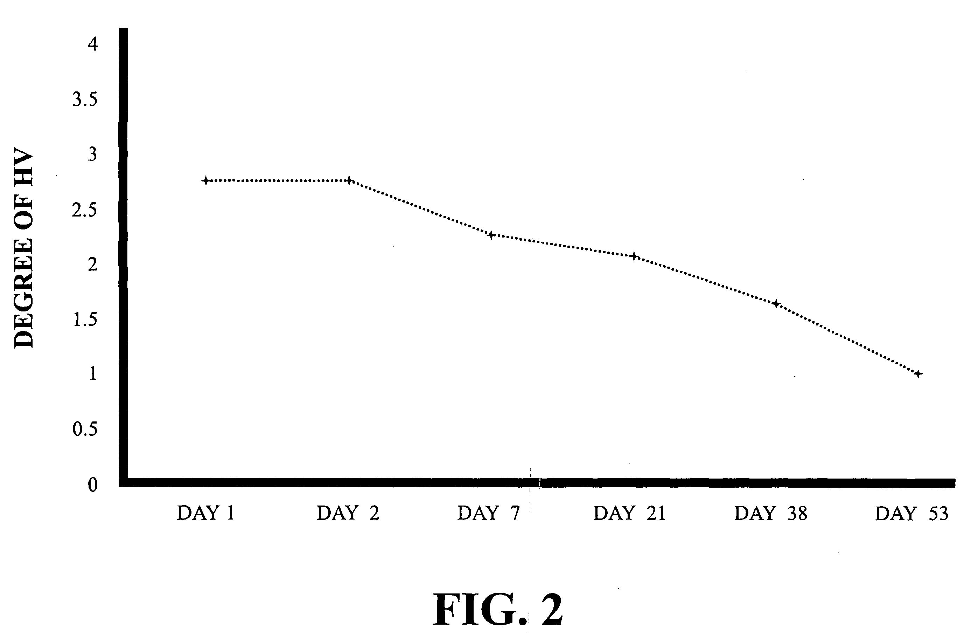

In the second experimental model (figure 2), on the seventh day after induction of the vitreous hemorrhage in the right eye of the rabbits, an intravitreous injection of a solution called AL025 was applied to them. This solution consisted of urea or carbamide. For this experimental model, it was observed that after the intravitreous injection of solution AL025 was applied, the vitreous hemorrhage began to diminish as of the third week of the study period. However, as is observed in the graph of figure 2, although the degree of the vitreous hemorrhage decreased, it remained above a degree greater than 1.0 at the end of the predetermined period of 53 days.

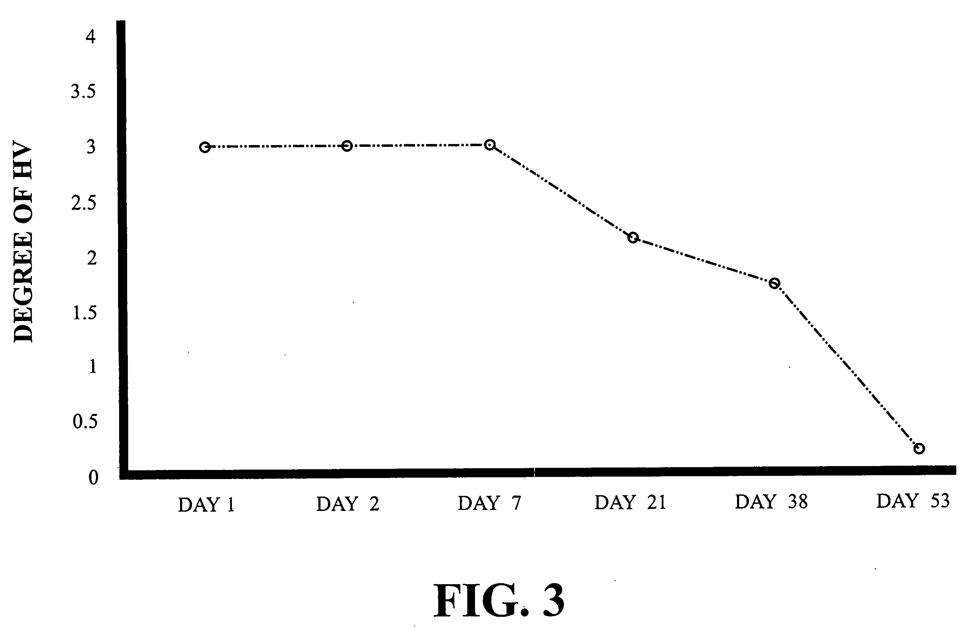

Referring now to figure 3, this corresponds to the graph of the experimental model developed for the treatment of the vitreous hemorrhage by injecting, into the eye of the species used, a therapeutically effective dose of the new ophthalmic solution, called here AL027, which encourages the reabsorption of the vitreous hemorrhage. As is observed in the behavior graph of the vitreous hemorrhage, after the application of the injection of solution AL027 on the seventh date, the degree of the hemorrhage began to gradually diminish until it reached a value of no more than 0.2 degrees of hemorrhage at the end of the preset 53-day study period.

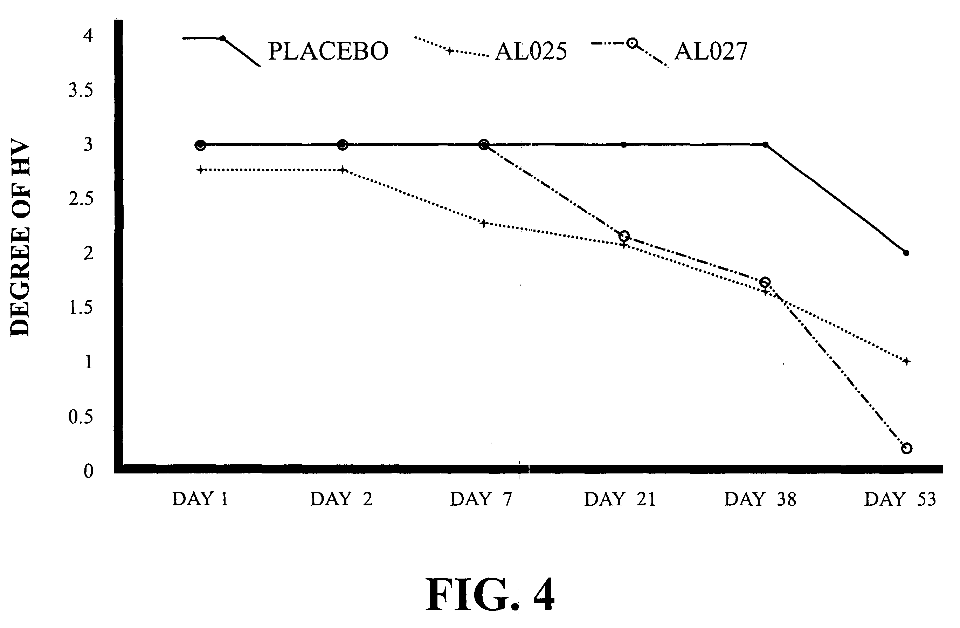

For its part, the graph of figure 4 represents the behavior of the vitreous hemorrhage after application of the injection in each experimental model described above. This graph enables a comparison to be made of the behavior of the hemorrhage in each model and as it shows, the intravitreous injection of solution AL027 by far surpasses those used in the conduct of this study.

The results obtained in the aforementioned models and other additional ones that have been carried out using the new ophthalmic solution have demonstrated its viability in the treatment for the clarification of vitreous hemorrhages. Consequently, the solution AL027 materializes a new therapeutic option for the effective and safe treatment of such hemorrhages.

The new ophthalmic solution for intravitreous injection is formulated essentially from a pharmaceutically effective amount of an active ingredient and a pharmaceutically acceptable quantity of a carrier solution; where the active ingredient is mannitol and its present in the solution at a sufficiently effective concentration to obtain the desired therapeutic effect. For example, the mannitol would be present in the new solution in a percentage of 5% to 30% in weight thereof.

Furthermore, the carrier solution can be Sophisen® and it would be present in the solution in a percentage that would range from 0.05% to 20% in weight. The ophthalmic solution Sophisen is protected under the Mexican patent No. 204310, European patent No. 0868 909 A2 and United States patent No. 6,071,958, all of which are owned by the same applicant. Additionally, the solution AL027 comprises 0.01% to 5% in weight of sodium phosphate monobasic monohydrate, 0.01% to 5% in weight of sodium phosphate dibasic anhydrous and injectable water in an appropriate proportion.

In an embodiment to exemplify the making of solution AL027 for clarification of vitreous hemorrhages, such solution comprises mannitol as the active ingredient in a quantity of 20 mg; 0.1 ml of Sophisen; 0.1 g of sodium phosphate monobasic monohydrate; 0.1 g of sodium phosphate dibasic anhydrous and 100 ml of injectable water. The pH of the solution obtained with the mixture of the aforementioned ingredients is in the range of 7.2 and the osmolarity of such solution is 1400 mOsm/kg.

The preceding formulation is proposed as an example of making the new injectable ophthalmic solution; however, it should in no way be construed in the limitative sense.

For all the above-described experimental models, it was decided to apply a single injection of the solution employed on the seventh day after induction of the vitreous hemorrhage. It was preferred not to administer additional injections during the treatment period due to the latent risk of provoking possible later lesions such as endophthalmitis, traumatic cataract or tears and separations of the retina during the injection process of the solutions applicable in each model.

Notwithstanding the foregoing, it is important to point out that the treatment method for the reabsorption of the vitreous hemorrhage may consist in administering more than one intravitreous injection of a pharmaceutically effective dose of the solution AL027, should it be necessary and therapeutically recommendable.

In each of the above-described therapeutic models, the intravitreous injection procedure was done as follows: after having induced the vitreous hemorrhage in the rabbits, with a preloaded insulin syringe armed with a 27 caliber needle, the sclera was penetrated at 3 mm from the sclerocorneal junction, above the superior temporal quadrant, aiming the needle toward the center of the vitreous cavity; once located here, 5 microliters of the solution employed in each experimental model were discharged and then the needle was withdrawn, exerting counterpressure at the site of the insertion of the needle with a sterile swab, and ophthalmic topical application of a solution of ciprofloxacin, as a prophylactic measure.

In addition to the evaluation and assessment made of the degree of vitreous hemorrhage, clinical aspects of safety were also evaluated, the results of which indicate that the ophthalmic solution AL027 is safe. For such purposes, the safety of AL027 was evaluated by electroretinogram and ultrasound B, and satisfactory results were obtained.

The preparation of the new AL027 solution can follow a process such as the one described hereunder:

Pour into a stainless steel recipient 800 ml of injectable water at a temperature of 40° ± 2°C; begin the agitation at 200 rpm ± 50 rpm and keep it constant throughout the entire preparation process; slowly add 200 g of an active ingredient, such as mannitol; cool the solution until it reaches a temperature of less than 35°C; add 1.0 g of sodium phosphate monobasic monohydrate; add 5.1 g of sodium phosphate dibasic anhydrous; add 1.0 ml of Sophisen; bring to a volume of 1 liter with injectable water; and agitate at 200 rpm ± 50 rpm until complete homogeneity is obtained.

With reference to the carrier solution Sophisen, this is constituted as follows: 10.20% in weight of polyoxyl stearate 40; 0.15% in weight of edetate disodium, dihydrate; 1.03% in weight of sodium chloride; 0.14% in weight of boric acid; 0.32% in weight of sorbic acid; 0.06% in weight of sodium bisulfate and 88.00% in weight of distilled water.

Although the above-described process leads to the obtainment of the ophthalmic solution, it is to be understood that there may be some other variants or processes that will lead to the formulation of such solution without straying from the inventive concept being claimed here. Thus, the foregoing process is only proposed by way of an example for making the new ophthalmic solution.

Although the invention has been described as above, in accordance with its form of manufacture or preferred embodiment, it is thought that some other variants and modifications may be derived from the know-how contained in the description or may be suggested by specialists in this matter. Consequently, it shall be understood that such alternatives of additional modifications would be considered as falling within the scope of the present invention, which is defined in the following claims.

## Claims

1. An intravitreous injectable solution for the treatment of vitreous hemorrhages, which comprises: a pharmaceutically effective quantity of an active ingredient; and a pharmaceutically acceptable quantity of a carrier solution.

2. The intravitreous injectable solution, according to claim 1, **characterized in that** said active ingredient is mannitol.

3. The intravitreous injectable solution, according to claim 1, **characterized in that** said carrier solution comprises: 10.20% in weight of polyoxyl stearate 40; 15% in weight of edetate disodium, dihydrate; 1.03% in weight of sodium chloride; 0.14% in weight of boric acid; 0.32% in weight of sorbic acid; 0.06% in weight of sodium bisulfate and 88.00% in weight of distilled water.

4. The intravitreous injectable solution, according to claim 1, **characterized in that** said carrier solution is Sophisen.

5. The intravitreous injectable solution, according to claim 1, **characterized in that** mannitol is present in a concentration that encourages the reabsorption of the vitreous hemorrhage.

6. The intravitreous injectable solution, according to claim 3, **characterized in that** the mannitol is present in the solution in a percentage of 5% to 30% in weight.

7. The intravitreous injectable solution, according to claim 1, **characterized in that** the Sophisen is present in the solution in a percentage of 0.02% to 20% in weight.

8. The intravitreous injectable solution, according to claim 1, **characterized in that** the pH of the solution is approximately 7.2.

9. The intravitreous injectable solution, according to claim 1, **characterized in that** the solution has an osmolarity of approximately 1400 mOsm/kg.

10. A method for the treatment of vitreous hemorrhages comprising: applying at least one injection of a pharmaceutically acceptable ophthalmic solution, into the vitreous humor of an eye with a hemorrhage resulting from a lesion or disease.

11. The method according to claim 10, **characterized by** applying at least one therapeutically effective dose of the injectable solution of claim 1, into the vitreous humor of a patient diagnosed with vitreous hemorrhage.

12. A method for the clarification of vitreous hemorrhages comprising the injection, into the vitreous body of the eye of a patient, of an ophthalmic solution, such as the one claimed in claim 1.

13. The method of the preceding claim, which is aimed at avoiding vitrectomy surgery in patients with vitreous hemorrhage, by the application of at least one intraocular injection of an ophthalmic solution formulated for the reabsorption of the hemorrhage.

14. The new use of mannitol as active ingredient of a solution that is injectable into the vitreous body of the eye for the treatment of vitreous hemorrhages.

15. A method for the preparation of an ophthalmic solution for an intravitreous injection for the treatment of vitreous hemorrhages, **characterized by** the following steps: pouring into a stainless steel recipient 800 ml of injectable water at a temperature of 40° ± 2°C; beginning the agitation at 200 rpm ± 50 rpm and keeping it constant throughout the entire preparation process; slowly adding 200 g of an active ingredient, such as mannitol; cooling the solution until it reaches a temperature of less than 35°C; adding 1.0 g of sodium phosphate monobasic monohydrate; adding 5.1 g of sodium phosphate dibasic anhydrous; adding 1.0 ml of Sophisen; bringing it to a volume of 1 liter with injectable water; and agitating at 200 rpm ± 50 rpm until complete homogeneity is obtained.

16. An intravitreous injectable solution for the treatment of vitreous hemorrhages comprising: a pharmaceutically effective quantity of mannitol: 0.01% to 5% in weight of sodium phosphate monobasic monohydrate; 0.01% to 5% in weight of sodium phosphate dibasic anhydrous and 100 ml of injectable water.

17. The intravitreous injectable solution of claim 16, in which the mannitol is present in a percentage of 5% to 30% in weight of the solution.

18. The intravitreous injectable solution of claim 16, which further includes 0.05% to 20% in weight of a carrier solution.

19. The intravitreous injectable solution of claim 16, wherein the carrier solution is Sophisen.
